# EUROPÄISCHE PATENTSCHRIFT

(11) **EP 1 132 102 B1**
(45) Veröffentlichungstag und Bekanntmachung des Hinweises auf die Patenterteilung: **03.09.2008**
(21) Anmeldenummer: 01250040.1
(22) Anmeldetag: 05.02.2001
(51) Int. Cl.: A61M 3/02, A61M 1/00, A61M 16/00, F04B 49/06, F04B 43/12

(54) **Medizintechnische Vorrichtung mit verschlüsselter Identinformation**
Medical device with encrypted identification
Appareil médical à identification cryptée

(30) Priorität: 07.02.2000 DE 10005108
(43) Veröffentlichungstag der Anmeldung: 12.09.2001
(73) Patentinhaber: W.O.M. World of Medicine AG, 96337 Ludwigsstadt (DE)
(72) Erfinder: Sasse, Joachim, 14476 Glienicke (DE); Stiller, Mathias, 14482 Potsdam (DE)
(74) Vertreter: Seuss, Thomas

(56) Entgegenhaltungen:
- EP-A- 0 771 569
- US-A- 4 865 321
- US-A- 4 897 789
- US-A- 5 460 490
- US-A- 5 647 854
- US-A- 5 714 060
- US-A- 5 730 720

## Beschreibung

Die Erfindung betrifft eine medizintechnische Vorrichtung mit einem medizintechnischen Gerät aufweisend einen Zubehöranschluß und mit zumindest einem Zubehörteil aufweisend ein zum Zubehöranschluß komplementäres Anschlußelement, nach dem Oberbegriff des Anspruchs 1. Eine solche Vorrichtung ist aus US 5,730,720 bekannt. Medizintechnische Vorrichtungen im Sinne der Erfindung sind insbesondere Saug- und/oder Spülpumpen oder Gasversorgungssysteme für die Bereiche Dialyse Arthroskopie, Hysteroskopie, Zystoskopie, Laparoskopie und Beatmungstechnik, wobei ein Zubehörteil typischerweise ein Schlauchset und/oder Filter, beispielsweise ein Pumpenschlauchset für Rollenpumpen, Gasschlauchsets, ggf. mit Sterilfilter und/oder Heizeinrichtung für ein durchgeleitetes Medium, zur Verbindung des medizintechnischen Gerätes mit einem ärztlichen Instrument für die besagten Anwendungsbereiche ist. Für ein Gerät kann eine Mehrzahl funktioneller Zubehörteile vorgesehen sein, wobei es sich versteht, daß bei Anwendung an einem einzigen Gerät bzw. Gerätetyp hinreichende mechanische Kompatibilität zwischen jeweiligem Anschlußelement und dem Zubehöranschluß besteht.

Eine medizintechnische Vorrichtung des eingangs genannten Aufbaus ist als interner Stand der Technik bekannt. Hierbei ist die Identinformation als Barcode gespeichert und wird mit einem Barcodeleser ausgelesen. Die Struktur von Barcodes ist bekannt und vereinheitlicht, so daß jeder beliebige Anwender einen Barcode auslesen kann. Insofern stellt ein üblicher Barcode nicht eine verschlüsselte Identinformation zur Verfügung; die Identinformation liegt gleichsam in Klartext vor.

Zubehörteile stellen in aller Regel Verbrauchsartikel dar, welche nur für eine begrenzte Zahl von Einsätzen geeignet und vorgesehen sind. Typischerweise sind viele Zubehörteile, insbesondere Schlauchsets aus Sterilitätsgründen Einmalartikel. Eine grundsätzliche Problematik bei medizintechnischen Geräten ist infolge dessen, daß Zubehörteile aus Kostengründen unzulässigerweise wiederverwendet werden könnten. Eine weitere Problematik ist, daß Anbieter von Zubehörteilen, und nur von Zubehörteilen, existieren, deren Produkte nicht allen Anforderungen, sei es in technischer oder sei es in medizinischer Hinsicht, genügen. Dies ist mit beachtlichen gesundheitlichen Risiken für Patienten verbunden, was aus ohne weiteres ersichtlichen Gründen stört.

Der vorstehenden Problematik ist von authorisierten Herstellern von Zubehörteilen mit verschiedenen Ansätzen gelöst worden. Bei Einsatz eines Barcodes liest der Barcodescanner den Barcode aus und überprüft, ob der gelesene Barcode mit einer Referenzliste zulässiger Barcodes korrespondiert. Bejahendenfalls kann eine Aktivierung der Vorrichtung erfolgten, verneinendenfalls nicht. Dieser interne Stand der Technik ist zwar ein Fortschritt zur Lösung der obigen Problematik, dennoch ist es verhältnismäßig leicht die insofern eingerichtete Sicherheitsfunktion zu umgehen. Denn ein Nachahmer braucht nur den Barcode auszulesen und auf seinen minderwertigen Produkten anzubringen.

Ein anderer Ansatz zu der genannten Problematik besteht in komplizierter Formgebung von Zubehöranschluß und Anschlußelement. Dies erschwert zwar die Herstellung von unauthorisierten Nachahmungen, führt aber andererseits zu einer beachtlichen Erhöhung des Aufwandes bei authorisierten Herstellern mit der störenden Folge höherer Preise für die Zubehörteile. Letztendlich wird kein Vorteil erreicht, da ein unauthorisierter Nachahmer dennoch auch vergleichsweise preisgünstig arbeiten kann durch medizinisch bedenkliche Einsparungen in den Bereichen der technischen Eigenschaften oder beispielsweise der Sterilität.

Weitere Problemkreise im Zusammenhang mit Zubehörteilen bestehen in der meist begrenzten Haltbarkeit und der damit verbundenen Verfallsdaten sowie der möglichen mißbräuchlichen Überschreitung der maximal zulässigen Anwendungszyklen (typischerweise 1 bei Schlauchsets). Diese werden durch die vorstehend beschriebenen Maßnahmen in keinster Weise gelöst.

Der Erfindung liegt daher das technische Problem zugrunde, eine medizintechnische Vorrichtung anzugeben, welche eine Gefährdung von Patienten durch Verwendung unzulässiger Zubehörteile ausschließt. Einer Weiterbildung der Erfindung liegt das weitere technische Problem zugrunde, eine Gefährdung von Patienten durch unzulässige Mehrfachverwendung von Zubehörteilen oder durch Verwendung von Zubehörteilen, deren Verfallsdatum überschritten ist, auszuschließen.

Zur Lösung des erstgenannten Problems lehrt die Erfindung eine medizintechnische Vorrichtung gemäß Anspruch 1. Als Speichereinheit ist jede beliebige Ausführungsform der Fixierung von Information bezeichnet. Hierunter fallen insbesondere elektronische, magnetische und optische Speichermedien. Im Rahmen der Erfindung kann eine Speichereinheit neben dem eigentlichen Speicher zusätzlich weitere Baugruppen aufweisen, beispielsweise Prozessoren und/oder Kommunikationsbaugruppen. Eine Ausleseeinheit umfaßt typischerweise neben den zur Auslesung notwendigen Kommunikationsbaugruppen auch elektronische Mittel zur Verarbeitung der ausgelesenen Identinformation, insbesondere zum Vergleich mit Identsollinformation und zur Ansteuerung des Geräts nach Maßgabe dieses Vergleichs. Identinformation bezeichnet eine nach Maßgabe des Aufbaus des eigentlichen Speichers aufbereitete Zeichenfolge, welche für einen bestimmten Typ eines Zubehörteils und ggf. auch für ein individuelles Zubehörteil charakteristisch ist und von einem authorisierten Hersteller vorgegeben wird. Falls die Identinformation individuell charakterisierend ist, empfiehlt es sich die Identinformation in eine Zubehörtypteilinformation und eine individuelle Teilinformation zu unterteilen. Dann wird der Vergleich mit der Identsollinformation typischerweise nur mit der Zubehörtypteilinformation durchgeführt. Der authorisierte Hersteller besorgt dann auch die Integration entsprechender Identinformation bzw. Zubehörtypteilinformation als Identsollinformation in die Ausleseeinheit. Verschlüsselte Identinformation unterscheidet sich von unverschlüsselter Identinformation dadurch, daß die unverschlüsselte Identinformation mittels des Schlüsselcodes transformiert worden ist. Auf binärer Ebene kann eine Verschlüsselung beispielsweise im einfachsten Fall durch Verschiebung der Bitwertigkeiten mittels einer Schieberegisterfunktion realisiert sein. Auf Ebene der ASCII Zeichen können beispielsweise mathematische Operationen bezüglich der alphabetischen Reihenfolge angebracht sein. Die vielen verschiedensten Verschlüsselungstechniken sind dem Fachmann gut vertraut und brauchen hier nicht näher erläutert zu werden. Eine verschlüsselte Identinformation im Sinne der Erfindung liegt aber auch dann vor, wenn die Identinformation an sich in der Speichereinheit in Klartext gespeichert ist, eine Auslesung der Speichereinheit jedoch nur mittels eines Schlüsselcodes möglich ist bzw. nicht rücklesbar ist (sogenannte verborgene Information).

Mit der Erfindung wird aufgrund der in der Speichereinheit verschlüsselt vorliegenden Identinformation und des zu Entschlüsseln (in der Speichereinheit oder der Ausleseeinheit) erforderlichen proprietären Schlüssels erreicht, daß eine Nachahmung auch der Speichereinheit einschließlich einer eine Inbetriebnahme der Vorrichtung erlaubenden Identinformation praktisch unmöglich, zumindest aber erheblich erschwert ist. Da nur authorisierte Hersteller für Zubehörteile den proprietären Schlüsselcode erhalten, ist eine Inbetriebnahme der Vorrichtung nur mit zugelassenen Zubehörteilen möglich. Bei Anschluß von nicht zugelassenen Zubehörteilen ist die Inbetriebnahme dagegen gesperrt und es empfiehlt sich, dann auch die Abgabe eines Alarmsignals, üblicherweise Ton oder Licht, zu aktivieren zwecks Aufmerksammachung auf das unzulässige Zubehörteil.

Die Erindung sieht die zwei folgenden Ausführungsformen vor. Zum ersten erfolgt die Entschlüsselung der Identinformation dadurch, daß die Ausleseeinheit den Schlüsselcode an die Speichereinheit übermittelt und die Speichereinheit bei Übereinstimmung des übermittelten Schlüsselcodes mit einem in der Speichereinheit gespeicherten Schlüsselcode die Identinformation in unverschlüsselter Form zur Auslesung freigibt. Dann ist die Identinformation in der Speichereinheit im Klartext, i.e. an sich unverschlüsselt, gespeichert. Zum zweiten erfolgt die Entschlüsselung der Identinformation dadurch, daß die Ausleseeinheit den Schlüsselcode an die Speichereinheit übermittelt und in der Speichereinheit mittels des Schlüsselcodes eine Transformation der verschlüsselten Identinformationen in unverschlüsselte Identinformation durchgeführt wird. Dann erfolgt eine Auslesung der unverschlüsselten Identinformation.

Von selbstständiger Bedeutung ist eine Ausführungsform der Erfindung, in welcher, ggf. unabhängig von den vorstehend erläuterten Merkmalen, die Ausleseeinheit (zusätzlich) ein in der Speichereinheit gespeichertes Verfallsdatum ausliest und mit dem in der Ausleseeinheit in Echtzeit mitlaufenden Datumswert vergleicht und bei Überschreitung des Verfallsdatums am Tage der Auslesung die Vorrichtung sperrt, und/oder in welcher Identinformation für ein Zubehörteil individualisierend ist und die Ausleseeinheit, bei erstmaligem Anschluß (oder Abtrennen) des Zubehörteils beginnend, die Anwendungszyklenzahl, bezogen auf die individuelle Teilinformation erfaßt und bei Überschreiten der maximal zulässigen Anwendungszyklenzahl die Vorrichtung sperrt. Letzteres kann im einzelnen dadurch erfolgen, daß die ausgelesene Zubehörtypteilinformation in der Ausleseeinheit mit einer in der Ausleseeinheit gespeicherten Tabelle verglichen wird, deren Elemente verschiedene Zubehörtypteilinformationen und zugeordnete maximale Anwendungszyklenzahlen sind. Nach Ermittlung der maximalen Anwendungszyklenzahl des angeschlossenen Zubehörteiltyps findet dann für die ebenfalls ausgelesene und abgespeicherte individuelle Teilinformation in der Ausleseeinheit ein Vergleich der tatsächlichen und der maximalen Anwendungszyklenzahl statt. In einer bevorzugten Variante enthält jedoch die Speichereinheit einen ersten Speicherzusatzbereich, in welchem die maximale Anwendungszyklenzahl abgespeichert ist, sowie einen zweiten Speicherzusatzbereich, in welchem die tatsächliche Anwendungszyklenzahl abgespeichert, wobei der Inhalt des zweiten Speicherzusatzbereichs bei jedem Anschluß (oder auch bei jedem Abtrennen) um eins erhöht wird, und wobei ein Vergleich beider Werte in der Speichereinheit oder der Auswerteeinheit (nach Auslesung beider Werte) erfolgt. Wesentliches Element dieser Ausführungsform ist demnach, daß die Ausleseeinheit auch in die Speichereinheit schreiben kann, i.e. Information in dem Speicherelement aufbauen oder verändern kann. Andere Varianten sind selbstverständlich ebenfalls möglich.

Mit dieser Ausführungsform wird erreicht, daß beispielsweise Einmal-Artikel, wie Einmal-Schlauchsets, nicht mehrmals verwendet werden bzw. daß Schlauchsets für die Mehrfachbenutzung nur der vorgebenen Anwendungszyklenzahl unterworfen werden. Zudem kann eine Verwendung eines an sich zulässigen Zubehörteils, dessen Verfallsdatum jedoch überschritten ist, verhindert werden.

Der Schlüsselcode sowie dessen Verarbeitung bzw. die Transformation der verschlüsselten Identinformation können auf alle üblichen Weisen ausgebildet sein. So ist es möglich, daß der Schlüsselcode ausgewählt ist aus der Gruppe bestehend aus "Festcode, Wechselcode, sequentieller Wechselcode, stochastischer Wechselcode, und Kombination solcher Codes". Bei einem Festcode ist der Schlüsselcode statisch. Bei einem sequentiellen Wechselcode wird der Schlüsselcode nach jeder Anwendung durch einen subsequenten neuen Schlüsselcode ersetzt. Subsequent können fortlaufende (z.B. zahlenmäßig fortlaufend oder echtzeitgebunden) Schlüsselcodes sein; hierunter fällt aber auch eine vorgewählte Reihenfolge inhaltlich nicht miteinander verknüpfter Schlüsselcodes einer vorgegebenen Schlüsselcodegruppe mit ggf. zyklischer Auswahl. Stochastische Wechselcodes werden zumindest zum Teil nach jeder Anwendung zufallsgeneratorgesteuert verändert. Bei allen Wechselcodes versteht es sich, daß Synchronizität zwischen Ausleseeinheit und Speichereinheit eingehalten werden muß. Wechselcodes verhindern Mißbrauch beispielsweise durch ein unerwünschtes "Abhören" des Schlüsselcodes bei einer Auslesung oder sonstigen Informationsübertragung zwischen Ausleseeinheit und Speichereinheit.

Im Einzelnen kann eine erfindungsgemäße Vorrichtung auf die verschiedensten Weisen ausgebildet sein. Es ist möglich, daß die Speichereinheit als Transponder und die Ausleseeinheit als Transponderabfragetransciever ausgebildet sind. Als Transponder wird ein elektronischer Schaltkreis bezeichnet, welcher auf Empfang eines Signals seinerseits Signale sendet. Ein Transponderabfragetransciever ist ein elektronischer Schaltkreis, welcher zur Aussendung eines einen Transponder aktivierenden Signals sowie zum Empfang eines Transponder-Signals eingerichtet ist. Die Begriffe "Senden" und "Empfangen" sind hierbei im allerweitesten Sinne verwendet. So kann die Informationsübertragung (u.a. Schlüsselcode, Identinformation usw.) zwischen Transponder und Transponderabfragetranciver mittels elektromagnetischer Wellen oder induktiv oder kapazitiv oder galvanisch erfolgen. Elektromagnetische Wellen sind insbesondere Funkwellen (beispielsweise UKW, VHF oder UHF Wellen) sowie Lichtwellen (beispielsweise IR, sichtbar, UV). Die einschlägigen Technologien sind gut bekannt und brauchen hier nicht näher erläutert zu werden. Eine galvanische Informationsübertragung erfolgt über mit Kontaktfeldern kontaktierte Kontaktgruppen (Chipkarten-Technologie). Es ist empfehlenswert, wenn mit einer Informationsübertragung von dem Transponderabfragetranciever zum Transponder auch zum Betrieb des Transponders benötigte Energie übertragen wird. Dann braucht der Transponder keine eigene Energieversorgung und die mit beispielsweise Batterien verbundene Entsorgungsproblematik wird vermieden.

Die Identinformation kann zusätzlich einen Kundencode enthalten, wodurch ein Austausch von Zubehörartikeln verhindert wird.

Die Erfindung umfaßt auch ein Verfahren zum Betrieb einer erfindungsgemäßen medizintechnischen Vorrichtung gemäß Anspruch 8. Im Zusammenhang mit diesen Verfahren gelten die vorstehenden Erläuterungen zur Vorrichtung entsprechend. Bei Einsatz programmierter Logik und/oder Prozessoren empfiehlt es sich, daß die Verarbeitungssoftware nicht rücklesbar ist, wodurch die Herstellung von Dubletten verhindert wird.

Im folgenden wird die Erfindung anhand von lediglich Ausführungsbeispiele darstellenden Figuren näher erläutert. Es zeigen:
- Fig. 1:: eine Teilansicht eines Zubehörteils
- Fig. 2:: eine Ansicht eines Gerätes, für welches der Gegenstand der Figur 1 bestimmt ist und
- Fig. 3:: ein Flußdiagramm für ein erfindungsgemäßes Verfahren.

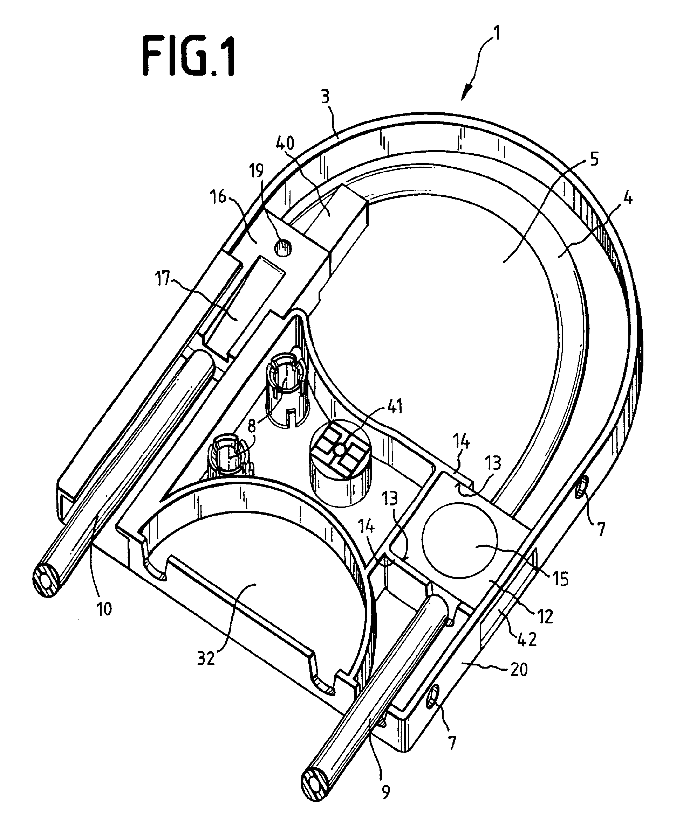

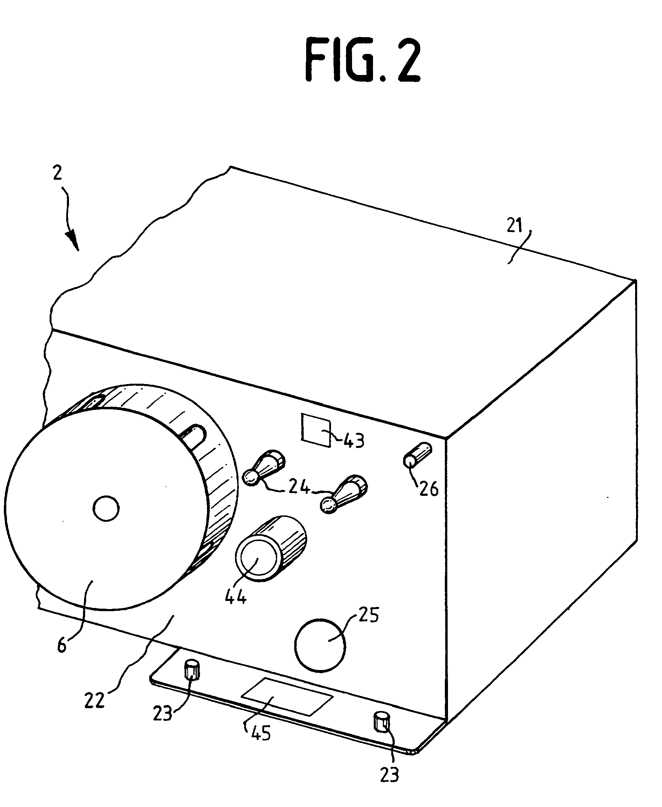

In der Fig. 1 erkennt man eine Schlauchkassette 1 für eine peristaltische Pumpe 2 (siehe auch Fig. 2) mit einem Kassettengehäuse 3, mit einem durch das Kassettengehäuse 3 verlaufenden flexiblen Schlauch 4, wobei der Schlauch 4 in dem Kassettengehäuse 3 entlang eines Kreissegments, im Ausführungsbeispiel ca. 180°, geführt ist. Das Kassettengehäuse 3 ist von in wesentlicher halbovaler Form mit zwei ebenen Halbovalflächen, wobei eine Halbovalfläche geschlossen und wobei die gegenüberliegende Halbovalfläche offen ist und somit eine Ausnehmung 5 zum Eingriff des Rollenrades 6 aufweist. Das Rollenrad 6 der peristaltischen Pumpe 2 greift bei montierter Schlauchkassette 1 in das Innere des Kreissegmentes ein. In der Fig. 1 erkennt man weiterhin Verbindungselemente 7, 8 zum Anschluß des Kassettengehäuses 3 an die peristaltische Pumpe 2. In der Fig. 2 ist zu erkennen, daß die das Rollenrad tragende Außenwandung 22 des Pumpengehäuses 21 zu den Verbindungselementen 7, 8 der Kassette komplementärer Anschlußelemente 23, 24 aufweist. In der Fig. 2 erkennt man weiterhin, daß das Rollenrad 6 über die Außenwandung 22 des Pumpengehäuses hervorragt. Bei montierter Schlauchkassette 1 ist die Drehachse des Rollenrades 6 im wesentlichen koaxial zum Kreissegment der Führung des Schlauches 4 in dem Kassettengehäuse 3, wie eine vergleichende Betrachtung der Fig. 1 und 2 verdeutlicht.

Der Fig. 1 entnimmt man, daß ein an das Kreissegment anschließender Schlauchschenkel 10 zwischen einer Montageposition und einer Betriebsposition verschieblich gelagert ist. Dargestellt ist die Betriebsposition. Man erkennt, daß ein Verbindungselement 16 mit einem Rastelement im Rahmen des verschieblichen Schlauchschenkels 10 eingerichtet ist. Das Rastelement ist mit einer Anlauffläche 17 ausgestattet, mittels welcher bei an der peristaltischen Pumpe 2 angebrachter Kassette 1 ein an der peristaltischen Pumpe 2 angeordnetes Sperrelement, ein Sperrstift, im Zuge der Verschiebung des verschieblichen Schlauchschenkels 10 aus der Montageposition in die Betriebsposition gegen Federdruck aus einer Ruhestellung aushebt. Das Rastelement weist in Richtung der Betriebsposition an die Anlauffläche 17 anschließend eine Sperrfläche auf, hinter welche das Sperrelement in seiner Sperrstellung greift. Im Rahmen der peristaltischen Pumpe 2 ist ein Schaltelement eingerichtet, welches mit dem Sperrstift wechselwirkt. Der Sperrstift kann in seiner Ruhestellung über seine Sperrstellung hinaus aus der Außenwandung 22 des Pumpengehäuses 21 hervortreten unter Krafteinwirkung einer Druckfeder. Hierbei ist durch geeignete Anordnung des Schaltelements ein Betätigungselement des Schaltelements freigegeben. Im Zuge der Verschiebung des Verbindungselementes 16 von der Montageposition in die Betriebsposition wird der Sperrstift gegen die Federkraft der Druckfeder in Richtung innerhalb der Außenwandung 22 des Pumpengehäuses 21 verschoben durch Einwirkung der Anlauffläche 17. Im Zuge dieser Bewegung wird das Betätigungselement des Schaltelements betätigt. Sobald die Betriebsposition erreicht ist, fällt der Sperrstift in eine Ausnehmung mit einer Sperrfläche 19 und somit in seine Sperrstellung. Hierdurch wird der Schlauch 4 entgegen seiner inneren elastischen Kräfte in der Betriebsposition gehalten. Von Bedeutung ist in diesen Zusammenhängen, daß der Sperrstift zusätzlich auf einem Grund der Ausnehmung aufschlägt, wobei Anordnung des Schaltelementes, Länge des Sperrstifts sowie Anordnung des Grundes mit der Maßgabe getroffen sind, daß das Betätigungselement in der Sperrstellung des Sperrstifts betätigt bleibt. Im Ergebnis ist über das Schaltelement das Rollenrad 6 nur in Betriebsposition des Schlauchschenkels 10 aktivierbar.

Der Fig. 1 entnimmt man, daß der zweite Schlauchschenkel 9 in Längsrichtung des Schlauches 4 fixiert ist. Im Rahmen des fixierten Schlauchschenkels 9 ist ein Verbindungselement 12 mit orthogonal zur Längserstreckung des Schlauches 4 verlaufenen Anschlagflächen 13 ausgebildet. Die Anschlagflächen 13 laufen gegen Fixierflächen 14 in dem Kassettengehäuse 1 an. Im Rahmen des Verbindungselements 12 des fixierten Schlauchschenkels 9 ist ein Drucksensor 15 eingerichtet, welcher bei an der peristaltischen Pumpe 2 angebrachter Kassette 1 mit der peristaltischen Pumpe 2 kommuniziert. Im Ausführungsbeispiel ist der Drucksensor 15 als Druckmembran 15 einer Druckkammer ausgebildet und am Pumpengehäuse 21 ist ein Druckwandler 25 mit der Maßgabe angebracht, daß die Druckmembran 15 bei montierter Kassette 1 am Druckwandler 25 anliegt (siehe auch Fig. 2). Der Fig. 1 entnimmt man, daß der Schlauch 4 einschließlich der Verbindungselemente 12, 16 dem Kassettengehäuse 3 entnehmbar ist. Die Entnahme erfolgt hierbei in Richtung etwa orthogonal nach oberhalb der Papierebene in der dargestellten Ansicht.

Der Schlauch 4 besteht im Bereich des Kreissegments bzw. zwischen den Verbindungselementen 12, 16 aus einem gummielestischen Werkstoff, nämlich Silikonkautschuk. Der Schlauch 4 kann gegenüberliegend anschließend an den Verbindungselementen 12, 16 aus dem gleichen oder einem hiervon verschiedenen Werkstoff bestehen. Insbesondere ist es möglich, daß insofern auch nicht gummielastische Werkstoffe verwendet werden.

Einer vergleichenden Betrachtung der Fig. 1 und 2 entnimmt man die Mittel zur Befestigung der Schlauchkassette 1 an der peristaltischen Pumpe 2. In der Fig. 1 ist ersichtlich, daß an einer orthogonal zu den Halbovalflächen stehenden Wandung 20 zwei Formschlußausnehmungen 7 eingerichtet sind, und daß in Abstand bezogen auf die Wandung 20 zu den Formschlußausnehmungen 7 zwei Kraftschlußverbindungselemente 8 eingerichtet sind. Im Rahmen der peristaltischen Pumpe sind komplementäre Anschlußelemente 23, 24 eingerichtet. Bei den Anschlußelementen 23 handelt es sich um in die Formschlußausnehmungen 7 formschlüssig eingreifende Haltestifte 23. Die Kraftschlußverbindungselemente 8 sowie die hierzu komplementären Anschlußnippel 24 sind als lösbare Kraftschlußverbindung ausgebildet. Zum Ansetzen der Schlauchkassette 1 an die peristaltische Pumpe 2 werden zunächst die Formschlußausnehmungen 7 auf die Haltestifte 23 positioniert und aufgeschoben, wobei die Schlauchkassette 1 gegenüber der Außenwandung 22 abgekippt gehalten wird. Es versteht sich, daß zwischen den Formschlußausnehmungen 7 und den Haltestiften 23 ein hierfür ausreichendes Spiel vorgesehen ist. Nach dem Aufsetzen wird die Schlauchkassette 1 gegen die Außenwandung 22 gekippt und angedrückt, wodurch die Verbindung zwischen den Kraftschlußverbindungselementen 8 und Anschlußnippeln 24 hergestellt und die Schlauchkassette 1 an der peristaltischen Pumpe 2 gehalten wird. Die Abnahme der Schlauchkassette erfolgt in umgekehrter Richtung.

Im Ausführungsbeispiel funktioniert der Schlauch 4 als Spülleitung. Ebenso kann der Schlauch 4 aber auch als Saugleitung funktionieren. Weiterhin ist es, unabhängig vom Ausführungsbeispiel, möglich, daß im Rahmen der Kassette ein zweiter Schlauch eingerichtet ist, beispielsweise als Ablaßleitung funktionierend, wobei im Rahmen der peristalitischen Pumpe Absperrmittel eingerichtet sein können, welche bei angesetzter Kassette den zweiten Schlauch absperren. Im Ausführungsbeispiel gemäß Fig. 1 ist hierzu eine Kassettenkammer 32 vorgesehen. Im übrigen wird zu weiteren Details ausdrücklich auf die Patentanmeldung DE-199 60 668 verwiesen.

In der Figur 1 erkennt man weiterhin verschiedene Ausführungsformen von Speichereinheiten. Man erkennt einen Magnetstreifen 40, einen Mikrochip 41 sowie einen Transponder 42. In der Figur 2 sind die entsprechenden, zugeordneten Ausleseeinheiten dargestellt. Man erkennt einen Magnetstreifenleser 43 für den Magnetstreifen 40, eine Vorrichtung 44 zur Kontaktierung des Mikrochips 41 und einen Transponderabfragetransciever 45 zum Informationsaustausch mit dem Transponder 42. Der Informationsaustausch mit dem Transponder erfolgt induktiv. Es versteht sich, daß die jeweilig zueinander gehörigen Komponenten in der Schlauchkassette 1 und an der peristaltischen Pumpe 2 so positioniert sind, daß Ausleseeinheit und Speichereinheit in der gewünschten Art und Weise miteinander kommunizieren können. Der Magnetstreifen 40 wird im Zuge der Bewegung des Verbindungselements 16 aus seiner Montageposition in seine Betriebsposition so an dem Magnetstreifenleser 43 vorbeigezogen, daß seine Identinformation ausgelesen wird. Der Mikrochip 41 wird bei Ansetzen der Schlauchkassette 1 kontaktiert. Der Transponder 42 wird bei dem Ansetzen der Schlauchkassette 1 in die Nähe des Transponderabfragtranscievers 45 gebracht mit der Maßgabe, daß auch die zum Betrieb des Transponders 42 benötigte Energie induktiv übertragbar ist. Im Falle des Mikrochips 41 und des Transponders 42 erfolgt eine Auslesung dadurch, daß mit der Betätigung des Schaltelements 26 erfindungsgemäße Programmabläufe, insbesondere eine Aktivierung der Ausleseeinheit zur Auslesung der Identinformation, initiiert werden.

Es versteht sich, daß die vorstehend in einer Schlauchkassette 1 beschriebenen Varianten von Speichereinheiten mit den zugehörigen Ausleseeinheiten nur aus Gründen der Einfachheit in einer einzelnen Schlauchkassette 1 bzw. einer einzelnen peristaltischen Pumpe 2 dargestellt sind, üblicherweise reicht eine Variante völlig aus zur Durchführung der Erfindung.

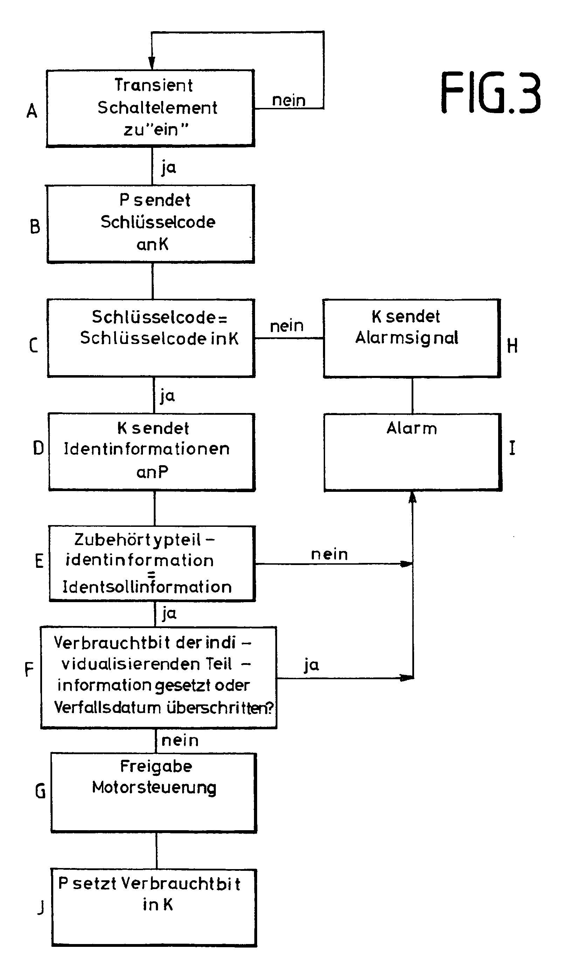

Mit dem Anschluß der Schlauchkassette 1 an die peristaltische Pumpe und der beschriebenen Initiierungsvorgänge werden die Routinen gemäß der Figur 3 eingeleitet. Anfangs überprüft das Programm in der Ausleseeinheit P der Pumpe 1, ob das Schaltelement 26 einen Transient zu "ein" meldet aufgrund des Ansetzens und Spannens einer Schlauchkassette 2. Sobald ein solcher Transient detektiert wird, sendet die Ausleseeinheit P einen Schlüsselcode an die Speichereinheit K der Kassette 1. In der Speichereinheit K wird dann der empfangene Schlüsselcode mit dem gespeicherten Schlüsselcode verglichen. Wenn keine Übereinstimmung besteht, so sendet die Speichereinheit K ein Alarmsignal an die Ausleseeinheit P mit der Folge, daß die Ausleseeinheit P einen Alarm der peristaltischen Pumpe 2 erzeugt. Bei Übereinstimmung sendet die Speichereinheit K die in ihr gespeicherte Identinformation an die Ausleseeinheit P. Die Ausleseeinheit P vergleicht dann die in der Identinformation enthaltene Zubehörtypteilidentinformation mit einer in der Ausleseeinehit P gespeicherten Liste zugelassener Zubehörtypteilidentsollinformation. Bei keiner Übereinstimmung wird von der Ausleseeinheit P der Alarm der persitaltischen Pumpe 2 aktiviert. Bei einer Übereinstimmung wird in der Ausleseeinheit P geprüft, ob ein Verbauchtbit, welches Teil der individualisierenden Teilinformation (Teil der bereits übermittelten Identinformation) ist, gesetzt ist oder ob ein weiterhin Teil der individualisierenden Teilinformation bildendes Verfallsdatum überschritten ist. Für letzteres enthält die Ausleseeinheit eine Echtzeituhr mit Datumsfunktion. Wenn eine der Bedingungen zutrifft, wird der Alarm aktiviert. Wenn keine der Bedingungen zutrifft, wird die Motorsteuerung zur Aktivierung beispielsweise durch eine Bedienperson freigegeben. Die Freigabe hält an bis zu einer Deaktivierung der Motorsteuerung, sei es durch eine Bedienperson oder durch einen Transient des Schaltelements 26 nach "aus". Nach der Freigabe sendet die Ausleseeinheit P ein Signal, mit welchem das Verbrauchtbit in der Speichereinheit gesetzt wird. Im Ergebnis kann kein nicht authorisiertes Zubehörteil verwendet werden und auch bereits gebrauchte oder überalterte authorisierte Zubehörteile sind von der Anwendung ausgeschlossen. Die Funktionen A, B, E, F, G, I und J (teilweise) sind in dem Programm der Ausleseeinheit implementiert. Die Funktionen C, D, H und J (teilweise) sind in dem Programm der Speichereinheit implementiert.

## Patentansprüche

1. Medizintechnische Vorrichtung mit einem medizintechnischen Gerät (2) aufweisend einen Zubehöranschluß und mit zumindest einem Zubehörteil (1) aufweisend ein zum Zubehöranschluß komplementäres Anschlußelement sowie einer im Bereich des Zubehöranschlusses angeordneten Ausleseeinheit (P),
wobei das Anschlußelement eine Speichereinheit (K) aufweist, in welcher entschlüsselbare Identinformation gespeichert ist,
wobei bei an dem Gerät (2) angeschlossenem Zubehörteil (1) Verschlüsselte oder unverschlüsselte Identinformation mittels der Ausleseeinheit (P) auslesbar ist,
wobei ausgelesene und entschlüsselte Identinformation mit in der Ausleseeinheit (P) gespeicherter Identsollinformation vergleichbar und die medizintechnische Vorrichtung bei Übereinstimmung zwischen Identinformation und Identsollinformation aktivierbar und bei nicht Übereinstimmung sperrbar ist,
**dadurch gekennzeichnet, daß** entweder
die Entschlüsselung des Identinformation **dadurch** erfolgt, daß die Ausleseeinheit (P) einen Schlüsselcode an die Speichereinheit (K) übermittelt und die Speichereinheit (K) bei Übereinstimmung des übermittelten Schlüsselcodes mit einem gespeicherten Schlüsselcode die Identinformation in ungeschlüsselter Form zur Auslesung freigibt
oder
die Entschlüsselung der Identinformation **dadurch** erfolgt, daß die Ausleseeinheit (P) einen Schlüsselcode an die Speichereinheit (K) übermittelt und in der Speichereinheit (K) mittels des Schlüsselcodes eine Transformation der verschlüsselten Identinformationen in unverschlüsselte Identinformation durchgeführt wird,
wobei in beiden Alternativen der Schlüsselcode proprietär ist.

2. Vorrichtung nach Anspruch 1, wobei der Schlüsselcode ausgewählt ist aus der Gruppe bestehend aus "Festcode, Wechselcode, sequentieller Wechselcode, stochastischer Wechselcode, und Kombination solcher Codes".

3. Vorrichtung nach Anspruch 1, wobei die Speichereinheit ferner einen ersten Speicherzusatzbereich enthält, in welchem die maximale Anwendungszyklenzahl abgespeichert ist, sowie einen zweiten Speicherzusatzbereich, in welchem die tatsächliche Anwendungszyklenzahl abgespeichert ist, wobei der Inhalt des zweiten Speicherzusatzbereichs bei jedem Anschluß oder auch bei jedem Abtrennen um eins erhöht wird, und wobei ein Vergleich beider Werte in der Speichereinheit oder der Auswerteeinheit nach Auslesung beider Werte erfolgt.

4. Vorrichtung nach einem der Ansprüche 1-3, wobei die Speichereinheit (K) als Transponder (42) und die Ausleseeinheit (P) als Transponderabfragetransceiver (45) ausgebildet sind.

5. Vorrichtung nach Anspruch 4, wobei eine Informationsübertragung zwischen Transponder (42) und Transponderabfragetransceiver (45) mittels elektromagnetischer Wellen oder induktiv oder kapazitiv oder galvanisch erfolgt.

6. Vorrichtung nach Anspruch 4 oder 5, wobei mit einer Informationsübertragung von dem Transponderabfragetransciever (45) zum Transponder (42) auch zum Betrieb des Transponders (42) benötigte Energie übertragen wird.

7. Vorrichtung nach einem der Ansprüche 1-6 in der Ausführungsform als Saug- und/oder Spülpumpe oder Gasversorgungssystem für die Bereiche Arthroskopie, Hysteroskopie, Zystoskopie, Laparoskopie und Beatmungstechnik, wobei das Zubehörteil ein Schlauchset zur Verbindung des medizintechnischen Gerätes mit einem ärztlichen Instrument für die besagten Bereiche ist.

8. Verfahren zum Betrieb einer Vorrichtung nach einem der Ansprüche 1-7, wobei nach Anschluß eines Zubehörteils an das Gerät die in der Speichereinheit (K) gespeicherte Identinformation, ggf. nach Entschlüsselung, ausgelesen wird, wobei ausgelesene und entschlüsselte Identinformation mit in der Ausleseeinheit (P) gespeicherter Identsollinformation verglichen wird und wobei die medizintechnische Vorrichtung bei Übereinstimmung zwischen Identinformation und Identsollinformation aktiviert und bei Nichtübereinstimmung gesperrt wird.

9. Verfahren zum Betrieb einer Vorrichtung nach Anspruch 3, wobei nach Anschluß eines Zubehörteils, welches nur eine begrenzte Anzahl von Anwendungszyklen zuläßt, an das Gerät die in der Speichereinheit (K) gespeicherte Identinformation, ggf. nach Entschlüsselung, ausgelesen wird, wobei ausgelesene und entschlüsselte Identinformation mit in der Ausleseeinheit (P) gespeicherter Identsollinformation verglichen wird, wobei die medizintechnische Vorrichtung bei Übereinstimmung zwischen Identinformation und Identsollinformation aktiviert und bei Nichtübereinstimmung gesperrt wird und wobei nach jedem Anschluß oder jeder Abtrennung die tatsächliche Anwendungszyklenzahl abgespeichert wird, wobei der Inhalt des zweiten Speicherzusatzbereichs bei jedem Anschluß oder auch bei jedem Abtrennen um eins erhöht wird.

## Claims

1. A medical device with a medical apparatus (2) comprising an accessory port, and at least one accessory piece (1) comprising a connection element being complementary to said accessory port and a readout unit (P) located in the area of the accessory port,
the connection element comprising a storage unit (K) wherein coded identification information is stored,
wherein, if the accessory piece (1) is connected to the apparatus (2), coded or un-coded identification information is readable by means of the readout unit (P),
wherein read-out and de-coded identification information is compared to preset valid identification information stored in the readout unit (P), and wherein the medical device is activated if the identification information is matching the preset valid identification information, and is blocked if the identification information does not match,
**characterized by** that either
decoding of the identification information is achieved by that the readout unit (P) transmits a key code to the storage unit (K), and that the storage unit (K) enables the identification information in an un-coded manner for readout, if the transmitted key code matches a stored key code,
or
decoding of the identification information is achieved by that the readout unit (P) transmits a key code to the storage unit (K), and that in the storage unit (K) a transformation of the coded identification information into un-coded identification information is performed by means of the key code,
in both alternatives the key code being proprietary.

2. The device according to claim 1, wherein the key code is selected from the group including "a fixed code, a changing code, a sequential changing code, a stochastic changing code, and combination of such codes".

3. The device according to claim 1, wherein the storage unit further comprises a first additional storage section, in which the maximum number of application cycles is stored, and a second additional storage section, in which the actual number of application cycles is stored, wherein the content of the second additional storage section is increased by one at every connection or also at every separation, and wherein a comparison of both values is made in the storage unit or the evaluation unit after readout of both values.

4. The device according to one of claims 1 to 3, wherein the storage unit (K) includes a transponder (42) and the readout unit (P) includes a transponder scanning transceiver (45).

5. The device according to claim 4, wherein a transmission of information between the transponder (42) and the transponder scanning transceiver (45) takes place by means of electro-magnetic waves or inductively or capacitively or galvanically.

6. The device according to claim 4 or 5, wherein with the transmission of information from the transponder scanning transceiver (45) to the transponder (42), the power required for operating the transponder (42) is also transmitted.

7. The device according to one of claims 1 to 6 in the embodiment as a sucking and/or rinsing pump or a gas supply system for the fields arthroscopy, hyteroscopy, cystoscopy, laparoscopy and breathing technologies, wherein the accessory piece is a hose set for the connection of the medical apparatus to a medical instrument for the mentioned fields.

8. A method for operating a device according to one of claims 1 to 7, wherein after connection of an accessory piece to the apparatus, the identification information stored in the storage unit (K) is read-out, if applicable after decoding, wherein read-out and decoded identification information is compared with the preset valid identification information stored in the readout unit (P), and wherein the medical apparatus is activated if the identification information is matching the preset valid identification information, and is blocked if the identification information does not match.

9. A method for operating a device according to claim 3, wherein after connection of an accessory piece, which permits a limited number of application cycles only, to the apparatus, the identification information stored in the storage unit (K) is read out, if applicable after decoding, wherein read-out and decoded identification information is compared with the preset valid identification information stored in the readout unit (P), wherein the medical device is activated if the identification information is matching the preset valid identification information, and is blocked if the identification information does not match, and wherein after every connection or every separation the actual number of application cycles is stored, wherein the content of the second additional storage section is increased by one at every connection or also at every separation.

## Revendications

1. Dispositif médical avec un appareil médical (2) comprenant un port d'accessoire, et au moins une pièce d'accessoire (1) comprenant un élément de connexion étant complémentaire audit port d'accessoire et une unité de lecture (P) positionnée dans la zone du port d'accessoire,
l'élément de connexion comprenant une unité de stockage (K), dans laquelle des informations d'identification codées sont emmagasinées,
dans lequel, si la pièce d'accessoire (1) est liée à l'appareil (2), des informations d'identification codées ou non-codées sont lisibles au moyen de l'unité de lecture (P),
dans lequel des informations d'identification lues et décodées sont comparées à des informations d'identification nominales emmagasinées dans l'unité de lecture (P), et dans lequel le dispositif médical est activé si les informations d'identification correspondent aux informations d'identification nominales, et est bloqué si les informations d'identification ne correspondent pas,
**caractérisé en ce que**
le décodage des informations d'identification est atteint par ce que l'unité de lecture (P) transmet un code clé à l'unité de stockage (K), et que l'unité de stockage (K) libère les informations d'identification d'une manière non-codée pour la lecture, si le code clé transmis correspond à un code clé emmagasiné,
ou
le décodage des informations d'identification est atteint par ce que l'unité de lecture (P) transmet un code clé à l'unité de stockage (K), et que dans l'unité de stockage (K) une transformation des informations d'identification codées en des informations d'identification non-codées est effectuée au moyen du code clé, dans les deux alternatives le code clé étant propriétaire.

2. Dispositif selon la revendication 1, dans lequel le code clé est choisi à partir d'un groupe comprenant «un code fixe, un code modifiable, un code modifiable séquentiel, un code modifiable stochastique, et des combinaisons de tels codes».

3. Dispositif selon la revendication 1, dans lequel l'unité de stockage en outre comprend une première section de mémoire additionnelle, dans laquelle le nombre maximum des cycles d'application est emmagasiné, et une deuxième section de mémoire additionnelle, dans laquelle le nombre actuel des cycles d'application est emmagasiné,
dans lequel le contenu de la deuxième section de mémoire additionnelle est augmenté de un à chaque connexion ou aussi à chaque séparation, et
dans lequel une comparaison des deux valeurs est effectuée dans l'unité de stockage ou l'unité d'évaluation après la lecture des deux valeurs.

4. Dispositif selon une des revendications 1 à 3, dans lequel l'unité de stockage (K) comprend un transpondeur (42) et l'unité de lecture (P) comprend un émetteur-récepteur de balayage (45).

5. Dispositif selon la revendication 4, dans lequel une transmission d'information entre le transpondeur (42) et l'émetteur-récepteur de balayage (45) a lieu au moyen d'ondes électromagnétiques ou de manière inductive ou capacitive ou galvanique.

6. Dispositif selon la revendication 4 ou 5, dans lequel avec la transmission d'information à partir de l'émetteur-récepteur de balayage (45) au transpondeur (42), l'énergie nécessaire pour l'opération du transpondeur (42) est aussi transmise.

7. Dispositif selon une des revendications 1 à 6 dans la forme d'exécution d'une pompe d'aspiration et/ou pompe de rinçage ou d'un système d'alimentation de gaz pour les domaines arthroscopie, hytéroscopie, cystoscopie, laparoscopie et technique respiratoire, dans lequel la pièce d'accessoire est un jeu de tuyaux pour la connexion de l'appareil médical à un instrument médical pour les domaines mentionnés.

8. Procédé pour la mise en service d'un dispositif selon une des revendications 1 à 7, dans lequel après la connexion d'une pièce d'accessoire à l'appareil, les informations d'identification emmagasinées dans l'unité de stockage (K) sont lues, le cas échéant après décodage, dans lequel les informations d'identification lues et décodées sont comparées avec les informations d'identification nominales emmagasinées dans l'unité de lecture (P), et dans lequel l'appareil médical est activé si les informations d'identification correspondent aux informations d'identification nominales, et est bloqué si les informations d'identification ne correspondent pas.

9. Procédé pour la mise en service d'un dispositif selon la revendication 3, dans lequel après la connexion d'une pièce d'accessoire, qui permet seulement un nombre limité de cycles d'application, à l'appareil, les informations d'identification emmagasinées dans l'unité de stockage (K) sont lues, le cas échéant après décodage, dans lequel les informations d'identification lues et décodées sont comparées avec les informations d'identification nominales emmagasinées dans l'unité de lecture (P), dans lequel le dispositif médical est activé si les informations d'identification correspondent aux informations d'identification nominales, et est bloqué si les informations d'identification ne correspondent pas, et dans lequel après chaque connexion ou chaque séparation le nombre actuel des cycles d'application est emmagasiné, dans lequel le contenu de la deuxième section de mémoire additionnelle est augmenté de un à chaque connexion ou aussi à chaque séparation.
